# EUROPEAN PATENT SPECIFICATION

(11) **EP 2 645 955 B1**
(45) Date of publication and mention of the grant of the patent: **26.10.2016**
(21) Application number: 11794353.0
(22) Date of filing: 30.11.2011
(51) Int. Cl.: A61B 18/14

(54) **EXPANDABLE ANGULAR VASCULAR ELECTRODE FOR RENAL NERVE ABLATION**
EXPANDIERBARE ABGEWINKELTE VASKULÄRE ELEKTRODE FÜR NIERENNERVENABLATION
ÉLECTRODE VASCULAIRE ANGULAIRE EXTENSIBLE POUR ABLATION DU NERF RÉNAL

(30) Priority: 30.06.2011 US 201161503378 P; 01.12.2010 US 418653 P; 15.12.2010 US 423439 P; 22.11.2011 US 201113302651; 23.09.2011 US 201113243724
(43) Date of publication of application: 09.10.2013
(73) Proprietor: Boston Scientific Scimed, Inc., Maple Grove, MN 55311 (US)
(72) Inventor: SMITH, Scott, Chaska MN 55318 (US); JENSON, Mark, L., Greenfield MN 55357 (US)
(74) Representative: Vossius & Partner Patentanwälte Rechtsanwälte mbB
(86) International application number: PCT/US2011/062668
(87) International publication number: WO 2012/075156

(56) References cited:
- WO-A2-2004/100813
- WO-A2-2008/003058
- US-A1- 2008 281 312

## Description

### SUMMARY

WO 2008/003058 A2 relates to methods and systems for thermally-induced renal neuromodulation. Thermally-induced renal neuromodulation may be achieved via direct and/or via indirect application of thermal energy to heat or cool neural fibers that contribute to renal function, or of vascular structures that feed or perfuse the neural fibers. In the methods and systems of WO 2008/003058 A2, parameters of the neural fibers, of non-target tissue, or of the thermal energy delivery element may be monitored via one or more sensors for controlling the thermally-induced neuromodulation. Protective elements may be provided to reduce a degree of thermal damage induced in the non-target tissues.

US 2008/0281312 A1 discloses an ablation therapy system and method for treating continuous atrial fibrillation. The therapy system includes a Multi-Channel RF Ablation Generator, an ECG interface, an assembly of at least three ablation catheters, and an ECG interface operably coupling and interfacing the catheters to both an ECG unit and the RF Ablation Generator.

WO 2004/100813 A2 describes a catheter having at least one multi-purpose lumen formed through the catheter terminating proximal a relatively complex-shaped distal portion thereof. The relatively complex-shaped distal portion comprises a looped portion having diagnostic- and/or ablation-type electrodes coupled thereto and an elongated diameter-adjusting member coupled proximal the distal end of the looped portion. The multi-purpose lumen may be used to alternately accommodate a variety of dedicated materials such as (i) a guide wire for initial deployment or later repositioning of the catheter, (ii) a volume or flow of a contrast media and the like, (iii) a deployable hollow needle or tube and the like used to biopsy adjacent tissue or dispense a therapeutic agent into a volume of tissue, and (iv) a cooling fluid, such as saline solution and the like dispensed at least during therapeutic tissue ablation procedures.

The present invention relates to an apparatus according to claim 1. Further preferred embodiments of the invention are defined in the dependent claims.

Devices and systems of the disclosure are directed to ablating target tissue of or adjacent a body vessel, chamber, cavity, or tissue structure using an angular electrode arrangement. Devices and systems of the disclosure are directed to ablating target tissue of or adjacent a body vessel, chamber, cavity, or tissue structure using an angular electrode arrangement supported by a self-deploying structure transformable between a low-profiled introduction configuration and a larger-profile deployed configuration. Devices and systems are directed to denervating tissues that contribute to renal sympathetic nerve activity using radiofrequency energy delivered to one or more electrodes of an angular electrode arrangement during ablation.

Embodiments of the invention are directed to ablation apparatuses that include a flexible elongated element having a proximal end, a distal end, and a length sufficient to access a target vessel of the body relative to a percutaneous access location. A self-deploying structure is provided at the distal end of the elongated element and transformable between a low-profile introduction configuration and a larger-profile deployed configuration. The self-deploying structure of the invention comprises a plurality of pre-set bends arranged to contact a wall of the target vessel at discrete circumferential and axial locations when in the deployed configuration such that the self-deploying structure assures a polygonal spiral configuration in the deployed configuration. An electrode arrangement is supported by the self-deploying structure and configured to deliver ablative energy to target tissue adjacent the target vessel wall when the self-deploying structure is in the deployed configuration. A conductor arrangement is coupled to the electrode arrangement and extending along the length of the elongated element.

In various embodiments, the electrode arrangement includes at least one electrode, and the self-deploying structure is configured to position the at least one electrode a predetermined distance away from the target vessel wall while the ablative energy is delivered to the target tissue. In some embodiments, each of the pre-set bends includes electrically insulating material, and a segment of the elongated element between at least some of the pre-set bends comprises an electrode surface of the electrode arrangement.

According to other embodiments, the electrode arrangement includes at least one electrode, and the self-deploying structure is configured to position the at least one electrode at the target vessel wall while the ablative energy is delivered to the target tissue. In various embodiments, at least some of the pre-set bends comprise an electrode surface.

The elongated member, according to the present invention, is configured to assume a polygonal spiral configuration when in the deployed configuration. An actuation wire can be coupled to the distal end of the apparatus to facilitate deployment and collapsing of the self-deploying structure.

In accordance with some embodiments, the elongated member includes at least five of the pre-set bends arranged at a predetermined pitch relative to one another to provide a pre-established relative axial and circumferential separation of ablation sites. The self-deploying structure can comprise a shape-memory slotted tube or a superelastic slotted tube configured as a flexible self-deploying structure. For example, the elongated member may comprise a polymeric tube, and the conductor arrangement may comprise an insulated slotted metal tube electrically coupled to the at least one electrode.

In some embodiments, the conductor arrangement includes a plurality of conductors, and adjacent electrodes or electrode surfaces of the electrode arrangement are separated by electrically insulating material. Each electrode or electrode surface can be coupled to one of the plurality of conductors so that each electrode or electrode surface can be energized independently.

In further embodiments, the conductor arrangement includes a plurality of conductors, and the electrode arrangement includes a plurality of electrodes electrically isolated from one another. One of the plurality of electrodes is coupled to one of the plurality of conductors, and the electrode arrangement is operable in a unipolar configuration or a bipolar configuration.

One or more temperature sensors can be provided at the electrode arrangement. A sheath can be used to facilitate delivery of the self-deploying structure and electrode arrangement at or into a target vessel. The sheath preferably has a lumen dimensioned to receive the apparatus with the self-deploying structure in the low-profile introduction configuration. The sheath lumen is further dimensioned for placement within the target vessel or ostium of the target vessel.

According to various embodiments, the target vessel comprises a renal artery of a patient, and the target tissue comprises perivascular renal nerve tissue adjacent the renal artery. In other embodiments, the apparatus is configured for deployment at or within other body structures, such as other body vessels, chambers, cavities, organs and other tissue structures.

These and other features can be understood in view of the following detailed discussion and the accompanying drawings.

### BRIEF DESCRIPTION OF THE DRAWINGS

Figure 1 is an illustration of a right kidney and renal vasculature including a renal artery branching laterally from the abdominal aorta;
Figures 2A and 2B illustrate sympathetic innervation of the renal artery;
Figure 3A illustrates various tissue layers of the wall of the renal artery;
Figures 3B and 3C illustrate a portion of a renal nerve;
Figure 4 illustrates computer modeling of heat distribution through a vessel wall generated by an RF electrode placed in contact with the inner wall of the vessel;
Figure 5 illustrates computer modeling of heat distribution through the same vessel wall generated by an RF electrode placed in a non-contacting relationship with the inner wall of the vessel in accordance with various embodiments;
Figure 6 shows an ablation catheter with an ablation region comprising several spaced-apart electrode elements deployed in a lumen of a renal artery in accordance with various embodiments;
Figure 7 shows several electrode elements situated on a catheter shaft in a spaced-apart relationship relative to a multiplicity of electrically non-conductive regions so that electrically non-conductive pre-set bends of the shaft, but not the electrode elements, make contact with the inner wall of the renal artery in accordance with various embodiments;
Figure 8A illustrates an electrode arrangement configured as an angular electrode having a flexible, self-expanding electrode spacing structure in accordance with various embodiments;
Figure 8B shows a portion of the spacing structure of figure 8A including hinge elements that provide for deployment into a preferred geometric shape;
Figure 9 shows the ablation region of a catheter deployed within a renal artery, with off-wall electrode elements positioned so that collectively, the electrode elements can create a substantially spiral lesion that includes outer adventitial and perivascular renal nerve tissue;
Figure 10 shows the distal end of an ablation catheter which includes an ablation region placed within a delivery sheath, the ablation region incorporating a flexible, self-deploying electrode spacing structure shown in a somewhat compressed state;
Figure 11 illustrates an ablation catheter which includes a flexible structure that assumes a generally basket shape and at least one electrode situated within the flexible basket-shaped structure;
Figures 12 and 13 illustrate an expandable angular electrode that provides for good vessel wall apposition and self-centering within a lumen of a target vessel in accordance with various embodiments; and
Figure 14 shows a representative renal denervation apparatus.

### DESCRIPTION

Embodiments of the disclosure are directed to apparatuses for ablating target tissue from within a vessel. Embodiments of the disclosure are directed to apparatuses for ablating perivascular renal nerves from within the renal artery for the treatment of hypertension. Embodiments of the disclosure are directed to a flexible structure of an ablation catheter configured to maintain positioning of one or more electrodes in a space-apart relationship relative to an inner wall of a vessel during renal nerve ablation. Embodiments of the disclosure are directed to a flexible structure of an ablation catheter configured to maintain positioning of one or more electrodes in a contacting or near-contacting relationship relative to an inner wall of a vessel during renal nerve ablation.

The present disclosure describes embodiments directed to off-wall positioning of one or more ablation electrodes within a target vessel of the body during ablation of nearby target tissue. The present disclosure also describes embodiments directed to on-wall or near-on-wall positioning of one or more ablation electrodes within a target vessel of the body during ablation of nearby target tissue.

According to various embodiments, a catheter has a distal RF ablation electrode arrangement and a positioning mechanism to hold the electrode(s) a controlled distance away from a target vessel wall, such as a wall of a renal artery. RF energy passes through the blood for a short distance before entering the artery wall and passing through the perivascular tissues to a ground pad. The current density in the artery wall is lower than in direct contact electrodes. Somewhat greater power is required because some of the energy passes through the blood and through other, non-target, tissues. However, cooling by arterial blood flow is greatly improved, especially at the artery wall which is covered by the electrode in direct contact electrode configurations, so that somewhat greater power delivery still results in a significantly cooler artery wall. Various positioning mechanisms can be used to accurately position the electrode a small distance from the artery wall.

Other embodiments of the disclosure are directed to an ablation device that has a relatively simple wire or tube construction, with an ablation region being pre-set to take a shape with multiple short bends. When released in the target vessel, such as a renal artery, by advancement of the device or by retraction of an external sheath, the ablation region of the device deploys to a polygonal spiral configuration. Shape-memory or superelastic slotted tube configurations can be utilized to provide a flexible self-deploying, self-centering multi-electrode structure. The self-deploying structure in preferably configured to hold multiple electrodes (although a single electrode embodiment is contemplated) in a contacting or near-contacting relationship relative to the wall of the target vessel during ablation.

Various embodiments of the disclosure are directed to apparatuses for renal denervation for treating hypertension. Hypertension is a chronic medical condition in which the blood pressure is elevated. Persistent hypertension is a significant risk factor associated with a variety of adverse medical conditions, including heart attacks, heart failure, arterial aneurysms, and strokes. Persistent hypertension is a leading cause of chronic renal failure. Hyperactivity of the sympathetic nervous system serving the kidneys is associated with hypertension and its progression. Deactivation of nerves in the kidneys via renal denervation can reduce blood pressure, and may be a viable treatment option for many patients with hypertension who do not respond to conventional drugs.

The kidneys are instrumental in a number of body processes, including blood filtration, regulation of fluid balance, blood pressure control, electrolyte balance, and hormone production. One primary function of the kidneys is to remove toxins, mineral salts, and water from the blood to form urine. The kidneys receive about 20-25% of cardiac output through the renal arteries that branch left and right from the abdominal aorta, entering each kidney at the concave surface of the kidneys, the renal hilum.

Blood flows into the kidneys through the renal artery and the afferent arteriole, entering the filtration portion of the kidney, the renal corpuscle. The renal corpuscle is composed of the glomerulus, a thicket of capillaries, surrounded by a fluid-filled, cup-like sac called Bowman's capsule. Solutes in the blood are filtered through the very thin capillary walls of the glomerulus due to the pressure gradient that exists between the blood in the capillaries and the fluid in the Bowman's capsule. The pressure gradient is controlled by the contraction or dilation of the arterioles. After filtration occurs, the filtered blood moves through the efferent arteriole and the peritubular capillaries, converging in the interlobular veins, and finally exiting the kidney through the renal vein.

Particles and fluid filtered from the blood move from the Bowman's capsule through a number of tubules to a collecting duct. Urine is formed in the collecting duct and then exits through the ureter and bladder. The tubules are surrounded by the peritubular capillaries (containing the filtered blood). As the filtrate moves through the tubules and toward the collecting duct, nutrients, water, and electrolytes, such as sodium and chloride, are reabsorbed into the blood.

The kidneys are innervated by the renal plexus which emanates primarily from the aorticorenal ganglion. Renal ganglia are formed by the nerves of the renal plexus as the nerves follow along the course of the renal artery and into the kidney. The renal nerves are part of the autonomic nervous system which includes sympathetic and parasympathetic components. The sympathetic nervous system is known to be the system that provides the bodies "fight or flight" response, whereas the parasympathetic nervous system provides the "rest and digest" response. Stimulation of sympathetic nerve activity triggers the sympathetic response which causes the kidneys to increase production of hormones that increase vasoconstriction and fluid retention. This process is referred to as the renin-angiotensin-aldosterone-system (RAAS) response to increased renal sympathetic nerve activity.

In response to a reduction in blood volume, the kidneys secrete renin, which stimulates the production of angiotensin. Angiotensin causes blood vessels to constrict, resulting in increased blood pressure, and also stimulates the secretion of the hormone aldosterone from the adrenal cortex. Aldosterone causes the tubules of the kidneys to increase the reabsorption of sodium and water, which increases the volume of fluid in the body and blood pressure.

Congestive heart failure (CHF) is a condition that has been linked to kidney function. CHF occurs when the heart is unable to pump blood effectively throughout the body. When blood flow drops, renal function degrades because of insufficient perfusion of the blood within the renal corpuscles. The decreased blood flow to the kidneys triggers an increase in sympathetic nervous system activity (i.e., the RAAS becomes too active) that causes the kidneys to secrete hormones that increase fluid retention and vasorestriction. Fluid retention and vasorestriction in turn increases the peripheral resistance of the circulatory system, placing an even greater load on the heart, which diminishes blood flow further. If the deterioration in cardiac and renal functioning continues, eventually the body becomes overwhelmed, and an episode of heart failure decompensation occurs, often leading to hospitalization of the patient.

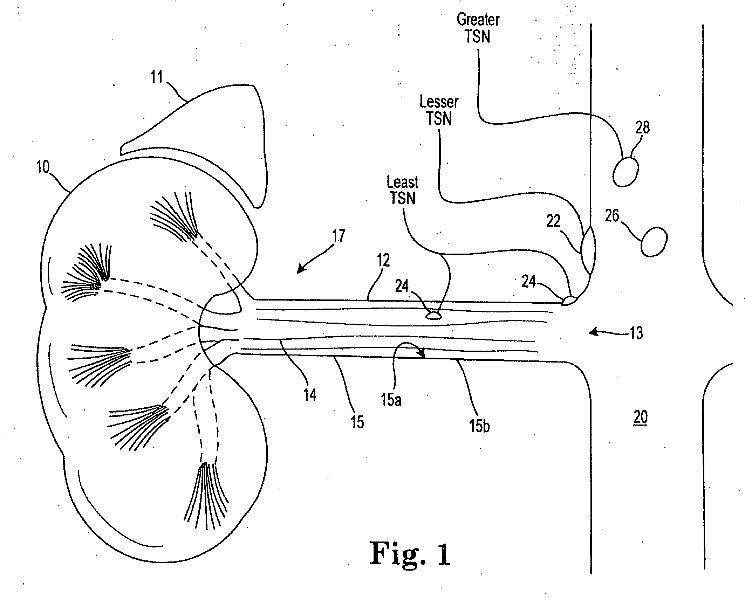

Figure 1 is an illustration of a right kidney 10 and renal vasculature including a renal artery 12 branching laterally from the abdominal aorta 20. In Figure 1, only the right kidney 10 is shown for purposes of simplicity of explanation, but reference will be made herein to both right and left kidneys and associated renal vasculature and nervous system structures, all of which are contemplated within the context of embodiments of the disclosure. The renal artery 12 is purposefully shown to be disproportionately larger than the right kidney 10 and abdominal aorta 20 in order to facilitate discussion of various features and embodiments of the present disclosure.

The right and left kidneys are supplied with blood from the right and left renal arteries that branch from respective right and left lateral surfaces of the abdominal aorta 20. Each of the right and left renal arteries is directed across the crus of the diaphragm, so as to form nearly a right angle with the abdominal aorta 20. The right and left renal arteries extend generally from the abdominal aorta 20 to respective renal sinuses proximate the hilum 17 of the kidneys, and branch into segmental arteries and then interlobular arteries within the kidney 10. The interlobular arteries radiate outward, penetrating the renal capsule and extending through the renal columns between the renal pyramids. Typically, the kidneys receive about 20% of total cardiac output which, for normal persons, represents about 1200 mL of blood flow through the kidneys per minute.

The primary function of the kidneys is to maintain water and electrolyte balance for the body by controlling the production and concentration of urine. In producing urine, the kidneys excrete wastes such as urea and ammonium. The kidneys also control reabsorption of glucose and amino acids, and are important in the production of hormones including vitamin D, renin and erythropoietin.

An important secondary function of the kidneys is to control metabolic homeostasis of the body. Controlling hemostatic functions include regulating electrolytes, acid-base balance, and blood pressure. For example, the kidneys are responsible for regulating blood volume and pressure by adjusting volume of water lost in the urine and releasing erythropoietin and renin, for example. The kidneys also regulate plasma ion concentrations (e.g., sodium, potassium, chloride ions, and calcium ion levels) by controlling the quantities lost in the urine and the synthesis of calcitrol. Other hemostatic functions controlled by the kidneys include stabilizing blood pH by controlling loss of hydrogen and bicarbonate ions in the urine, conserving valuable nutrients by preventing their excretion, and assisting the liver with detoxification.

Also shown in Figure 1 is the right suprarenal gland 11, commonly referred to as the right adrenal gland. The suprarenal gland 11 is a star-shaped endocrine gland that rests on top of the kidney 10. The primary function of the suprarenal glands (left and right) is to regulate the stress response of the body through the synthesis of corticosteroids and catecholamines, including cortisol and adrenaline (epinephrine), respectively. Encompassing the kidneys 10, suprarenal glands 11, renal vessels 12, and adjacent perirenal fat is the renal fascia, e.g., Gerota's fascia, (not shown), which is a fascial pouch derived from extraperitoneal connective tissue.

The autonomic nervous system of the body controls involuntary actions of the smooth muscles in blood vessels, the digestive system, heart, and glands. The autonomic nervous system is divided into the sympathetic nervous system and the parasympathetic nervous system. In general terms, the parasympathetic nervous system prepares the body for rest by lowering heart rate, lowering blood pressure, and stimulating digestion. The sympathetic nervous system effectuates the body's fight-or-flight response by increasing heart rate, increasing blood pressure, and increasing metabolism.

In the autonomic nervous system, fibers originating from the central nervous system and extending to the various ganglia are referred to as preganglionic fibers, while those extending from the ganglia to the effector organ are referred to as postganglionic fibers. Activation of the sympathetic nervous system is effected through the release of adrenaline (epinephrine) and to a lesser extent norepinephrine from the suprarenal glands 11. This release of adrenaline is triggered by the neurotransmitter acetylcholine released from preganglionic sympathetic nerves.

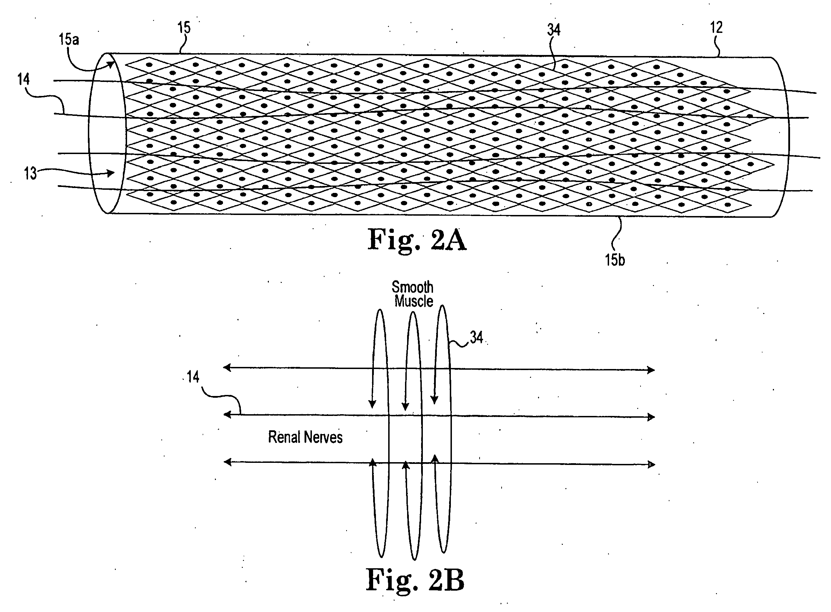

The kidneys and ureters (not shown) are innervated by the renal nerves 14. Figures 1 and 2A-2B illustrate sympathetic innervation of the renal vasculature, primarily innervation of the renal artery 12. The primary functions of sympathetic innervation of the renal vasculature include regulation of renal blood flow and pressure, stimulation of renin release, and direct stimulation of water and sodium ion reabsorption.

Most of the nerves innervating the renal vasculature are sympathetic postganglionic fibers arising from the superior mesenteric ganglion 26. The renal nerves 14 extend generally axially along the renal arteries 12, enter the kidneys 10 at the hilum 17, follow the branches of the renal arteries 12 within the kidney 10, and extend to individual nephrons. Other renal ganglia, such as the renal ganglia 24, superior mesenteric ganglion 26, the left and right aorticorenal ganglia 22, and celiac ganglia 28 also innervate the renal vasculature. The celiac ganglion 28 is joined by the greater thoracic splanchnic nerve (greater TSN). The aorticorenal ganglia 26 is joined by the lesser thoracic splanchnic nerve (lesser TSN) and innervates the greater part of the renal plexus.

Sympathetic signals to the kidney 10 are communicated via innervated renal vasculature that originates primarily at spinal segments T10-T12 and L1. Parasympathetic signals originate primarily at spinal segments S2-S4 and from the medulla oblongata of the lower brain. Sympathetic nerve traffic travels through the sympathetic trunk ganglia, where some may synapse, while others synapse at the aorticorenal ganglion 22 (via the lesser thoracic splanchnic nerve, i.e., lesser TSN) and the renal ganglion 24 (via the least thoracic splanchnic nerve, i.e., least TSN). The postsynaptic sympathetic signals then travel along nerves 14 of the renal artery 12 to the kidney 10. Presynaptic parasympathetic signals travel to sites near the kidney 10 before they synapse on or near the kidney 10.

With particular reference to Figure 2A, the renal artery 12, as with most arteries and arterioles, is lined with smooth muscle 34 that controls the diameter of the renal artery lumen 13. Smooth muscle, in general, is an involuntary non-striated muscle found within the media layer of large and small arteries and veins, as well as various organs. The glomeruli of the kidneys, for example, contain a smooth muscle-like cell called the mesangial cell. Smooth muscle is fundamentally different from skeletal muscle and cardiac muscle in terms of structure, function, excitation-contraction coupling, and mechanism of contraction.

Smooth muscle cells can be stimulated to contract or relax by the autonomic nervous system, but can also react on stimuli from neighboring cells and in response to hormones and blood borne electrolytes and agents (e.g., vasodilators or vasoconstrictors). Specialized smooth muscle cells within the afferent arteriole of the juxtaglomerular apparatus of kidney 10, for example, produces renin which activates the angiotension II system.

The renal nerves 14 innervate the smooth muscle 34 of the renal artery wall 15 and extend lengthwise in a generally axial or longitudinal manner along the renal artery wall 15. The smooth muscle 34 surrounds the renal artery circumferentially, and extends lengthwise in a direction generally transverse to the longitudinal orientation of the renal nerves 14, as is depicted in Figure 2B.

The smooth muscle 34 of the renal artery 12 is under involuntary control of the autonomic nervous system. An increase in sympathetic activity, for example, tends to contract the smooth muscle 34, which reduces the diameter of the renal artery lumen 13 and decreases blood perfusion. A decrease in sympathetic activity tends to cause the smooth muscle 34 to relax, resulting in vessel dilation and an increase in the renal artery lumen diameter and blood perfusion. Conversely, increased parasympathetic activity tends to relax the smooth muscle 34, while decreased parasympathetic activity tends to cause smooth muscle contraction.

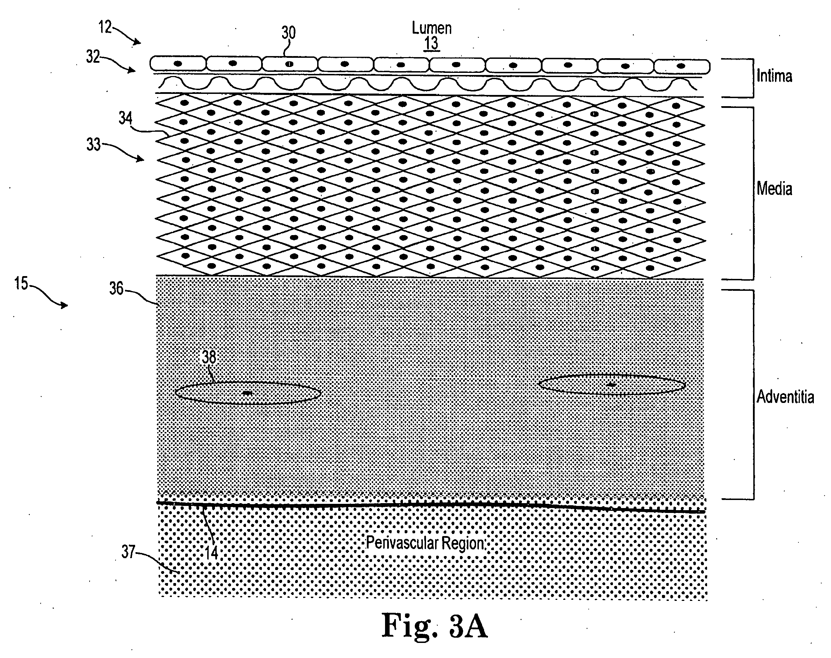

Figure 3A shows a segment of a longitudinal cross-section through a renal artery, and illustrates various tissue layers of the wall 15 of the renal artery 12. The innermost layer of the renal artery 12 is the endothelium 30, which is the innermost layer of the intima 32 and is supported by an internal elastic membrane. The endothelium 30 is a single layer of cells that contacts the blood flowing though the vessel lumen 13.
Endothelium cells are typically polygonal, oval, or fusiform, and have very distinct round or oval nuclei. Cells of the endothelium 30 are involved in several vascular functions, including control of blood pressure by way of vasoconstriction and vasodilation, blood clotting, and acting as a barrier layer between contents within the lumen 13 and surrounding tissue, such as the membrane of the intima 32 separating the intima 32 from the media 34, and the adventitia 36. The membrane or maceration of the intima 32 is a fine, transparent, colorless structure which is highly elastic, and commonly has a longitudinal corrugated pattern.

Adjacent the intima 32 is the media 33, which is the middle layer of the renal artery 12. The media is made up of smooth muscle 34 and elastic tissue. The media 33 can be readily identified by its color and by the transverse arrangement of its fibers. More particularly, the media 33 consists principally of bundles of smooth muscle fibers 34 arranged in a thin plate-like manner or lamellae and disposed circularly around the arterial wall 15. The outermost layer of the renal artery wall 15 is the adventitia 36, which is made up of connective tissue. The adventitia 36 includes fibroblast cells 38 that play an important role in wound healing.

A perivascular region 37 is shown adjacent and peripheral to the adventitia 36 of the renal artery wall 15. A renal nerve 14 is shown proximate the adventitia 36 and passing through a portion of the perivascular region 37. The renal nerve 14 is shown extending substantially longitudinally along the outer wall 15 of the renal artery 12. The main trunk of the renal nerves 14 generally lies in or on the adventitia 36 of the renal artery 12, often passing through the perivascular region 37, with certain branches coursing into the media 33 to enervate the renal artery smooth muscle 34.

Embodiments of the disclosure may be implemented to provide varying degrees of denervation therapy to innervated renal vasculature. For example, embodiments of the disclosure may provide for control of the extent and relative permanency of renal nerve impulse transmission interruption achieved by denervation therapy delivered using a treatment apparatus of the disclosure. The extent and relative permanency of renal nerve injury may be tailored to achieve a desired reduction in sympathetic nerve activity (including a partial or complete block) and to achieve a desired degree of permanency (including temporary or irreversible injury).

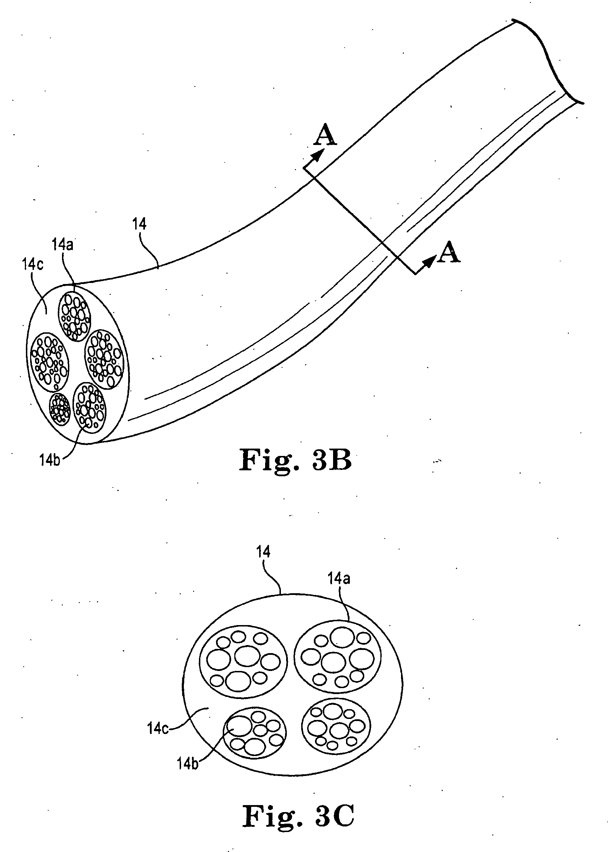

Returning to Figures 3B and 3C, the portion of the renal nerve 14 shown in Figures 3B and 3C includes bundles 14a of nerve fibers 14b each comprising axons or dendrites that originate or terminate on cell bodies or neurons located in ganglia or on the spinal cord, or in the brain. Supporting tissue structures 14c of the nerve 14 include the endoneurium (surrounding nerve axon fibers), perineurium (surrounds fiber groups to form a fascicle), and epineurium (binds fascicles into nerves), which serve to separate and support nerve fibers 14b and bundles 14a. In particular, the endoneurium, also referred to as the endoneurium tube or tubule, is a layer of delicate connective tissue that encloses the myelin sheath of a nerve fiber 14b within a fasciculus.

Major components of a neuron include the soma, which is the central part of the neuron that includes the nucleus, cellular extensions called dendrites, and axons, which are cable-like projections that carry nerve signals. The axon terminal contains synapses, which are specialized structures where neurotransmitter chemicals are released in order to communicate with target tissues. The axons of many neurons of the peripheral nervous system are sheathed in myelin, which is formed by a type of glial cell known as Schwann cells. The myelinating Schwann cells are wrapped around the axon, leaving the axolemma relatively uncovered at regularly spaced nodes, called nodes of Ranvier. Myelination of axons enables an especially rapid mode of electrical impulse propagation called saltation.

In some embodiments, a treatment apparatus of the disclosure may be implemented to deliver denervation therapy that causes transient and reversible injury to renal nerve fibers 14b. In other embodiments, a treatment apparatus of the disclosure may be implemented to deliver denervation therapy that causes more severe injury to renal nerve fibers 14b, which may be reversible if the therapy is terminated in a timely manner. In preferred embodiments, a treatment apparatus of the disclosure may be implemented to deliver denervation therapy that causes severe and irreversible injury to renal nerve fibers 14b, resulting in permanent cessation of renal sympathetic nerve activity. For example, a treatment apparatus may be implemented to deliver a denervation therapy that disrupts nerve fiber morphology to a degree sufficient to physically separate the endoneurium tube of the nerve fiber 14b, which can prevent regeneration and re-innervation processes.

By way of example, and in accordance with Seddon's classification as is known in the art, a treatment apparatus of the disclosure may be implemented to deliver a denervation therapy that interrupts conduction of nerve impulses along the renal nerve fibers 14b by imparting damage to the renal nerve fibers 14b consistent with neruapraxia. Neurapraxia describes nerve damage in which there is no disruption of the nerve fiber 14b or its sheath. In this case, there is an interruption in conduction of the nerve impulse down the nerve fiber, with recovery taking place within hours to months without true regeneration, as Wallerian degeneration does not occur. Wallerian degeneration refers to a process in which the part of the axon separated from the neuron's cell nucleus degenerates. This process is also known as anterograde degeneration. Neurapraxia is the mildest form of nerve injury that may be imparted to renal nerve fibers 14b by use of a treatment apparatus according to embodiments of the disclosure.

A treatment apparatus may be implemented to interrupt conduction of nerve impulses along the renal nerve fibers 14b by imparting damage to the renal nerve fibers consistent with axonotmesis. Axonotmesis involves loss of the relative continuity of the axon of a nerve fiber and its covering of myelin, but preservation of the connective tissue framework of the nerve fiber. In this case, the encapsulating support tissue 14c of the nerve fiber 14b is preserved. Because axonal continuity is lost, Wallerian degeneration occurs. Recovery from axonotmesis occurs only through regeneration of the axons, a process requiring time on the order of several weeks or months. Electrically, the nerve fiber 14b shows rapid and complete degeneration. Regeneration and re-innervation may occur as long as the endoneural tubes are intact.

A treatment apparatus may be implemented to interrupt conduction of nerve impulses along the renal nerve fibers 14b by imparting damage to the renal nerve fibers 14b consistent with neurotmesis. Neurotmesis, according to Seddon's classification, is the most serious nerve injury in the scheme. In this type of injury, both the nerve fiber 14b and the nerve sheath are disrupted. While partial recovery may occur, complete recovery is not possible. Neurotmesis involves loss of continuity of the axon and the encapsulating connective tissue 14c, resulting in a complete loss of autonomic function, in the case of renal nerve fibers 14b. If the nerve fiber 14b has been completely divided, axonal regeneration causes a neuroma to form in the proximal stump.

A more stratified classification of neurotmesis nerve damage may be found by reference to the Sunderland System as is known in the art. The Sunderland System defines five degrees of nerve damage, the first two of which correspond closely with neurapraxia and axonotmesis of Seddon's classification. The latter three Sunderland System classifications describe different levels of neurotmesis nerve damage.

The first and second degrees of nerve injury in the Sunderland system are analogous to Seddon's neurapraxia and axonotmesis, respectively. Third degree nerve injury, according to the Sunderland System, involves disruption of the endoneurium, with the epineurium and perineurium remaining intact. Recovery may range from poor to complete depending on the degree of intrafascicular fibrosis. A fourth degree nerve injury involves interruption of all neural and supporting elements, with the epineurium remaining intact. The nerve is usually enlarged. Fifth degree nerve injury involves complete transection of the nerve fiber 14b with loss of continuity.

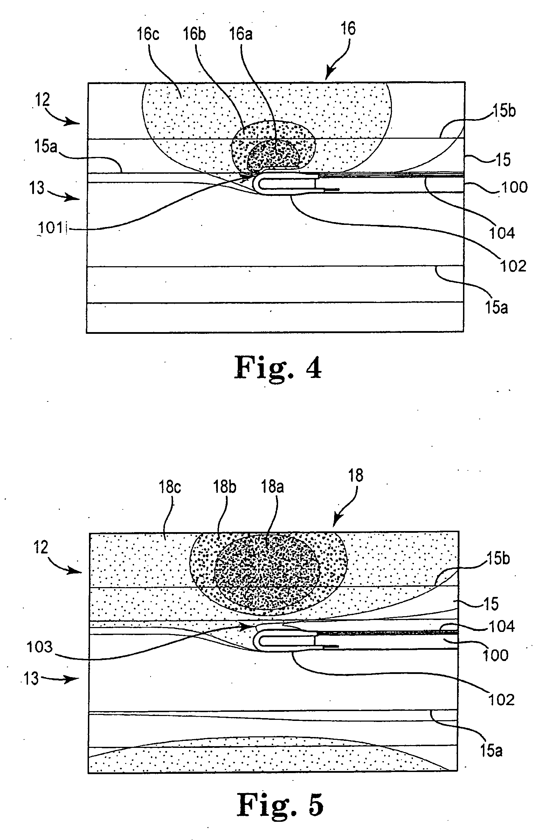

Turning now to Figures 4 and 5, there is illustrated computer modeling of heat distribution through a vessel wall generated by an RF electrode situated within the lumen of the vessel. In Figure 4, the electrode is situated in direct contact with the inner wall of the vessel. In Figure 5, the electrode is situated in a non-contacting (off-wall) relationship with respect to the inner wall of the vessel in accordance with various embodiments. It is readily apparent that temperatures of the vessel's inner wall resulting from the non-contact electrode-to-tissue interface shown in Figure 5 are significantly lower than vessel inner wall temperatures resulting from the direct contact electrode-to-tissue interface of Figure 4.

Figure 4 shows the heat distribution profile 16 within the renal artery wall 15 produced by an ablation electrode 102 of a catheter 100 placed in direct contact with the vessel's inner wall 15a. The catheter 100 includes a conductor 104 that runs the length of the catheter and is connected to the electrode 102 situated at the distal end of the catheter 100. In the configuration shown in Figure 4, the relative positioning of the electrode 102 and inner wall 15a of the renal artery wall 15 defines a direct contact electrode-to-tissue interface 101.

The heat distribution profile 16 of Figure 4 demonstrates that the artery wall tissue at or nearest the artery's inner wall 15a is subject to relatively high temperatures. As can be seen in Figure 4, heat produced by the electrode 102 at the direct contact electrode-to-tissue interface 101 is greatest at the inner wall 15a and decreases as a function of increasing distance away from the electrode 102. For example, a heating zone 16a associated with the highest temperatures produced by the ablation electrode 102 extends nearly the entire thickness of the artery wall 15. A zone 16b of lower temperatures relative to zone 16a extends radially outward from zone 16a, beyond the outer wall 15b of the renal artery 12, and into perivascular space adjacent the renal artery 12. A relatively cool zone 16c is shown extending radially outward relative to zones 16a and 16b. In this simulation, zone 16a is associated with temperatures required to ablate tissue which includes renal arterial wall tissue and perivascular renal nerves.

Figure 5 shows the ablation electrode 102 of catheter 100 situated in a spaced-apart relationship relative to the inner wall 15a of the renal artery 12. In the configuration shown in Figure 5, the relative positioning of the electrode 102 and inner wall 15a of the renal artery wall 15 defines a non-contact electrode-to-tissue interface 103. As can be seen in Figure 5, the heat distribution profile 18 differs significantly from that shown in Figure 4.

Importantly, zone 18a, which is associated with the highest temperatures (ablation temperatures), is translated or shifted outwardly away from the inner wall 15a and towards the outer wall 15b and perivascular space adjacent the renal artery 12. Zone 18a encompasses an outer portion of the adventitia of the renal artery wall 15 and encompasses a significant portion of the perivascular space adjacent the renal artery 12. As such, renal nerves and ganglia included within the adventitial tissue and perivascular space are subject to ablative temperatures, while the endothelium at the inner wall 15a of the renal artery 12 is maintained at a temperature which does not cause permanent injury to the inner wall tissue.

Off-wall electrode configurations according to various embodiments can reduce the RF current density in the artery wall 15, as the current spreads out somewhat as it passes between the electrode 102 and the artery wall 15 through the blood. This provides a sort of fluidic "virtual electrode" and results in less heating of the artery wall 15 due to the lower current density. According to various embodiments, structures that hold one or more electrodes at a prescribed distance away from the artery wall 15 preferably provide for passive cooling of the artery wall 15 during ablation by blood flowing through the artery 12. Separating the electrode(s) from the artery wall 15 by a structure that allows blood to pass along the artery wall 15 provides more effective cooling of the artery wall 15 (and the electrode 102), further reducing thermal injury to the artery wall 15. The current density in the target perivascular tissue can also be somewhat decreased, but the cooler artery wall temperatures allow greater overall power to be delivered safely, in order to achieve sufficient current density in the target tissue to ablate the target tissue.

Various embodiments of the disclosure are directed to apparatuses and methods for RF ablation of perivascular renal nerves for treatment of hypertension, with multiple electrodes held slightly away from the artery wall, decreasing current density, and improving cooling from the blood to reduce arterial injury while maintaining target tissue at ablation temperatures. Prior approaches have used an RF electrode placed in direct contact with the renal artery, but have had difficulty in repositioning the electrode to complete ablation while minimizing injury to the artery wall due to peak current density and heating at the wall contact points.

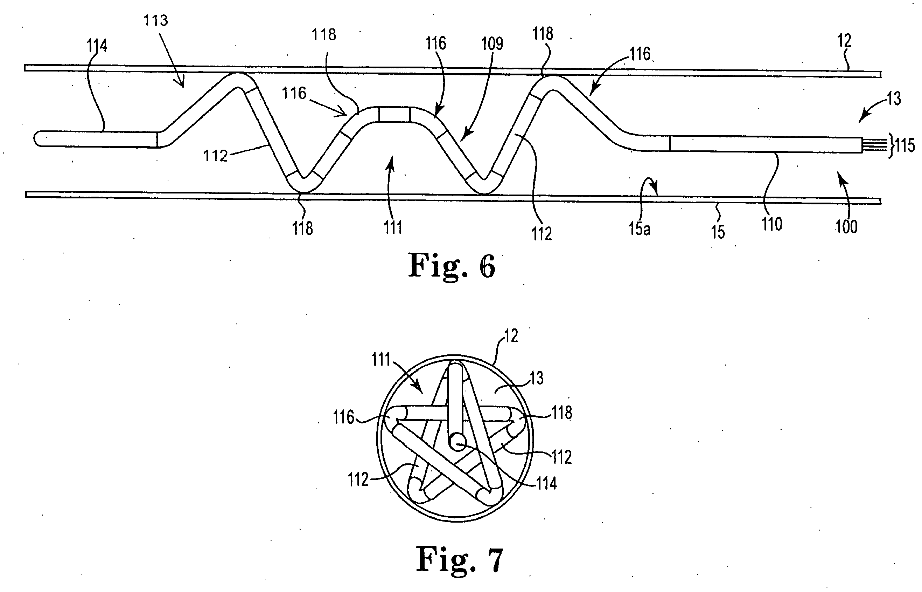

According to some embodiments, and with reference to Figures 6-10, an ablation apparatus may have a relatively simple wire or tube construction, with an ablation region preset to take a shape with multiple short bends. When released in the renal artery, such as by retraction of an external sheath, the ablation region deploys to a predefined configuration, such as a polygonal spiral configuration. Each bend contacts the artery's inner wall at discrete locations separated by circumferential and/or axial space, with connecting segments that span between the bends to create chords between the contact points. Each contacting bend portion has an electrically insulated surface, and each chord segment has a conductive electrode surface. The ablation apparatus can easily deploy to position multiple electrodes spaced circumferentially and axially apart within the lumen of the renal artery.

RF energy passes through the blood flowing within the renal artery for a short distance before entering the artery wall and passing through the perivascular tissues to an external ground pad. The current density in the renal artery wall is reduced and cooling of the artery wall by blood flow is greatly improved, resulting in a significantly cooler artery wall.

Shape-memory or superelastic slotted tube configurations can be utilized to provide a flexible, self-deploying electrode spacing structure. In various embodiments, the ablation apparatus can be fabricated of layers of tubing to electrically isolate a central lumen and the outer surface except for the electrode regions.

The electrodes can be energized simultaneously to rapidly provide multiple discrete ablations. A single insulated conductor can be used to conduct energy to the electrodes. Conductive portions of the tube or wire structure can be used to conduct the energy to the electrodes. In some embodiments, each electrode can be energized in sequence to reduce overall power requirements and reduce the required cooling from the blood. In this case, multiple insulated conductors can be provided.

Insulated conductor(s) can be routed through the central lumen of a slotted tube, for example. The spanning chord portions with the electrodes can have central insulated portions, so that only the portions within a desired range of distance from the artery wall function as electrodes. For example, only the portions of the chords that are 1 to 1.5 mm from the artery wall may be functioning electrodes, with other portions insulated.

In other embodiments, a polymer tube is utilized, with conductive electrode portions. A shape-memory or superelastic metal shaping component can be used to achieve the desired shaping. In some configurations, various numbers of electrodes can be used, such as 3 to 8. A 5-pointed configuration may have particular advantage in deploying to a useful configuration, with a certain "precession angle" or pitch to provide desired relative axial and circumferential separation of ablation sites. The lengths and number of segments are chosen to provide the desired separation between ablation sites in a relatively short space while accommodating some degree of anatomical variation.

In some configurations, a single electrode can be used, or other small number, while the self-deploying angular structure optimally locates the electrode spaced apart from the artery wall. In this case, the device can be moved and re-deployed to place the electrode(s) at different locations. In some embodiments, unipolar configurations can be used. In other embodiments, bipolar configurations can be used by energizing selected pairs of electrodes.

Thermocouples or other sensors can be incorporated in accordance with various embodiments. For example, a number of thermocouples can be distributed within the electrode arrangement or on the electrodes. Alternatively, thermocouples can be located at the bend sections so they contact the artery wall for measuring the artery wall temperature. The thermocouples can provide site-specific temperature information and be used for automatic ablation control.

Referring to Figure 6, there is shown an ablation catheter 100 with an ablation region 111 comprising several spaced-apart electrode elements 112 deployed in a lumen 13 of a renal artery 12 in accordance with various embodiments. The ablation region 111 includes an off-wall electrode arrangement 109 in accordance with various embodiments. The ablation region 111 is provided at a distal end of the elongated shaft 110 of the catheter 100. Although not shown, the shaft 110 is preferably a flexible elongated member having a proximal and, a distal end, and a length sufficient to access a patient's renal artery relative to a percutaneous access location.

The ablation region 111 is situated at the distal end of the elongated shaft 110. The ablation region 111 includes a flexible structure 113 configured to maintain an electrode arrangement 109 in a spaced relationship relative to an inner wall 15a of the renal artery 12 when in a deployed configuration. A flexible guide tip 114 is provided at the distal end of the ablation region 111 to aid in advancing the electrode arrangement 109 into the lumen 13 of the renal artery 12.

In the embodiment shown in Figure 6, the electrode arrangement 109 includes a number of electrode elements 112. Each of the electrode elements 112 is coupled to a conductor arrangement 115 and configured to deliver energy sufficient to ablate perivascular renal nerve tissue. In some embodiments, each electrode element 112 is coupled to an individual conductor of the conductor arrangement 115, allowing for energizing and de-energizing of individual electrode elements 112. At least one electrode element 112 is situated between adjacent electrically non-conductive regions 116 of the shaft 110. As shown in Figure 6, each of the electrically non-conductive regions 116 includes a pre-set bend 116 when the ablation region 111 is in a deployed configuration within the lumen 13 of the renal artery 12.

As is best seen in Figure 7, the spaced-apart electrode elements 112 are arranged on the shaft 110 relative to the electrically non-conductive regions 116 so that the pre-set bends 118 make contact with the inner wall 15a of the renal artery 12. The electrode elements 112 are maintained a distance away from the artery's inner wall 15a when the ablation region 111 is in its deployed configuration during and ablation procedure. A separation distance between the electrode elements 112 and the artery's inner wall 15a is preferably selected to provide efficient transfer of RF energy from the electrode elements 112 to arterial and perivascular renal nerve tissue with reduced injury to the endothelium when compared to a direct contact electrode-to-tissue interface. For example, a non-contact electrode-to-tissue interface provides for the transfer of heat generated by the electrode elements 112 to renal nerves and ganglia at or near the outer wall of the renal artery 12 and within the adjacent perivascular space with only negligible injury imparted to the endothelium layer of the renal artery 12.

According to some embodiments, the flexible structure 113 includes a shape-memory member or a super elastic member configured as a self-deploying electrode spacing structure. For example, the flexible structure 113 may include a multiplicity of electrically non-conductive regions configured to contact the inner wall 15a of the renal artery 12 at discrete circumferential locations when in a deployed configuration. The electrically non-conductive pre-set bends 118 are arranged so that the pre-set bends 118 contact the inner wall 15a of the renal artery 12 at the discrete circumferential locations. As can be seen in Figure 7, each of the electrode elements 112 is situated between adjacent pre-set bends 118 and are maintained in a spaced-apart relationship with respect to the artery's inner wall 15a by the geometry of the flexible structure 113 and spacing relationship between the electrode elements 112 and the pre-set bends 118 of the flexible structure 113.

In some embodiments, the flexible member 113 assumes a polygonal spiral configuration when deployed. In the deployed configuration, the pre-set bends 118 are spaced circumferentially and axially apart from one another on the expanded flexible member 113. According to one embodiment, the flexible member 113 includes at least five of the pre-set bends 113 arranged at a predetermined pitch relative to one another to provide a pre-established relative axial and circumferential separation of ablation sites.

The flexible member 113, according to various embodiments, comprises a shape-memory slotted tube or a super elastic slotted tube configured as a flexible, self-deploying electrode spacing structure. For example, the flexible member 113 may include a central lumen and a multiplicity of layers of to being that electrically isolate the central lumen and an outer surface of the flexible member 113 other than regions defining the electrode elements 112.

In other embodiments, the flexible member 113 defines a distal portion of a polymer tube of the elongated shaft 110 of the catheter 100 elongated shaft 110 of the catheter 100. A shape-memory for super elastic metal shaping member may be situated in the polymer to define the ablation region 111, which facilitates the ablation region assuming the spiral configuration within the deployed configuration. In other embodiments, the conductor arrangement 115 comprises an insulated slotted metal tube electrically coupled to the electrode elements 112. In some embodiments, insulated conductors can be passed through the central lumen of a tubular construction and attached to the electrode elements 112.

In embodiments that do not incorporate a self-deploying flexible structure 113, an actuation wire 119 may be coupled to the distal end of the catheter 100 to facilitate deployment and collapsing of the flexible structure. For example, an actuation wire 119 can be attached to the distal portion of the catheter 100, and push-pull actuation used to deploy and collapse the structure when desired. In embodiments that incorporate a self-deploying flexible structure 113, inclusion of an actuation wire 119 can be longitudinally advanced and retracted to assist in collapsing and expansion of the flexible structure 113.

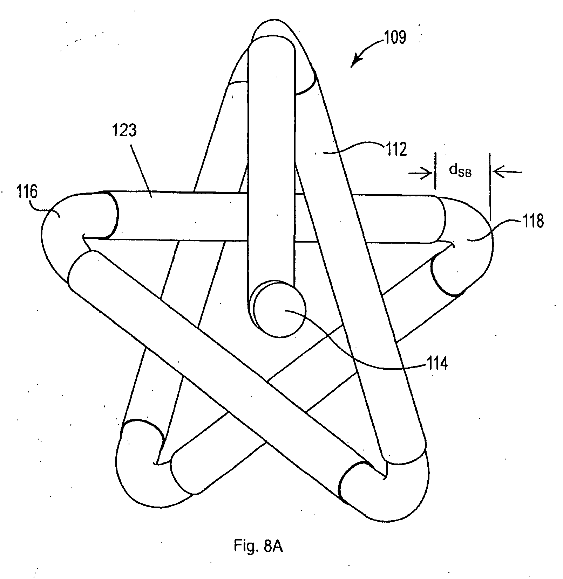

The electrode arrangement shown in Figure 8A is configured as an angular electrode which can be constructed as a hollow multi-channel or solid serial electrode. The open configuration of the electrode arrangement provides for passive cooling of the electrode elements by blood that flows through the vessel in which the electrode arrangement is deployed. A catheter shaft that supports the off-wall electrode arrangement 109 shown in Figure 8A may include a heat sink shaft/tip, such that the shaft adjacent to the tip assists with cooling. In this case, an elongated heat sink region of the tip can improve vessel wall cooling. In other embodiments, a non-streaming wet electrode can be implemented, which includes one or more weeping electrodes with a remote, low voltage exit that provides cooling and eschar resistance without stray heating. A reference electrode can be provided in accordance with other embodiments near the tip to accurately measure local power delivery.

In some configurations, a single electrode can be used, or other small number, while the self-deploying angular structure optimally locates the electrode spaced apart from the artery wall. In this case, the device can be moved and re-deployed to place the electrode(s) at different locations. In some embodiments, unipolar configurations can be used. In other embodiments, bipolar configurations can be used by energizing selected pairs of electrodes.

The embodiment shown in Figure 8A includes five spanning chords 123 defining the ablation region 111 of the flexible structure 113. Each of the five spanning chords 123 includes at least one electrode element 112 positioned between electrically non-conductive pre-set bends 118. Each of the electrode elements 112 is positioned a distance, d_{SB}, away from the portion of the pre-set bend 118 configured to contact the inner wall 15a of the renal artery 12. Preferably, the setback distance, d_{SB}, ranges from about 0.5 mm to about 2 mm. More preferably, the setback distance, d_{SB}, ranges from about 1 mm to about 1.5 mm.

It is noted that the number of electrode elements 112 can vary, typically between 3 and 8 electrode elements 112 (e.g. a 5-pointed configuration of a type discussed above). The length and the number of electrode segments preferably selected to provide a desired separation between ablation sites in a relatively short space while accommodating some anatomical variation.

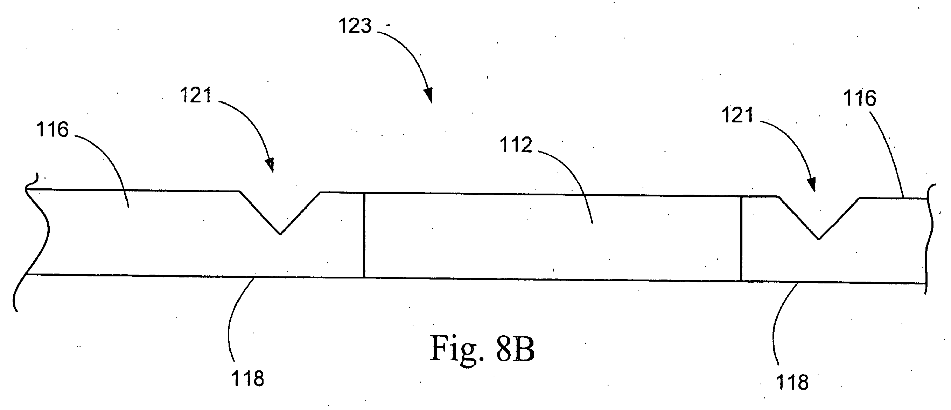

Referring now to Figure 8B, a portion of the off-wall electrode arrangement 109 shown in Figure 8A is illustrated. Figure 8B shows a spanning chord 123 which supports an electrode 112 and includes a pair of a hinges 121 adjacent opposing ends of the electrode 112. Each of the hinges 121 is configured to facilitate preferential bending of the spanning chord structure so as to define pre-set bends 118 of the off-wall electrode arrangement 109. In some embodiments, the spanning chords 123 can be fabricated from a superelastic slotted tube with a plastic material connected between adjacent slotted tubes to define a hinge. For example, and as shown in Figure 8B, the hinges 121 are formed as polymeric living hinges. In other embodiments, adjacent superelastic slotted tubes can be connected using separate flexible components, such as separate superelastic wires. In some embodiments, the hinges are smaller-diameter segments of the wire or tube.

Various implementations may be used to provide desired bending characteristics of the pre-set bends 118 of the off-wall electrode arrangement 109. Suitable hinges include those that bend easily in one plane, such hinges are referred to as orthotropic flexural stiffness hinges. A superelastic slotted tube represents a suitable structure for incorporating a hinge with desired orthotropic flexural stiffness characteristics. Other suitable hinges include orthotropic composite tubs, tubes with axial stiffeners, flat ribbons, bifilar arrangements of tubes, and multi-lumen tubing with lumens generally aligned with flexural plane.

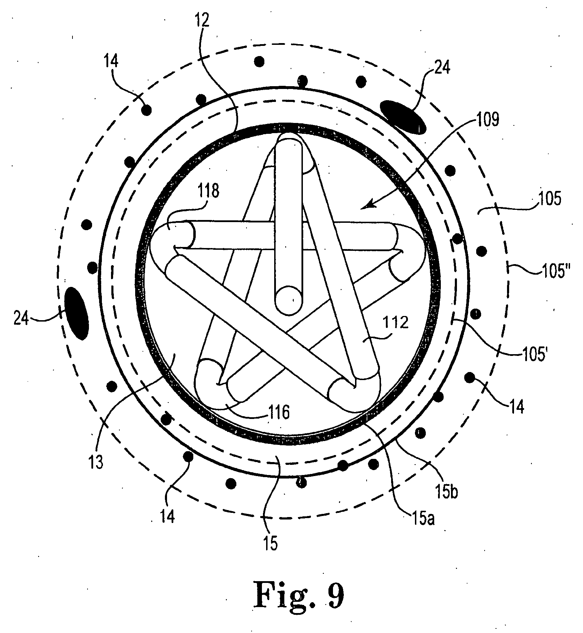

With reference to Figure 9, the ablation region 111 of the catheter 100 is shown deployed within the lumen 13 of the renal artery 12. In its deployed configuration, each of the preset-bends 118 contacts the inner wall 15a of the renal artery 12 at discrete locations which are spaced apart both circumferentially and axially. In this arrangement, the electrode elements 112 are positioned so that collectively, the electrode elements 112 can create a substantially spiral lesion that includes outer adventitial and perivascular renal nerve tissue. As is shown in Figure 9, an ablation zone 105 is treated by delivering sufficient RF power to the electrode elements 112, which are setback from the inner wall 15a of the renal artery 12. The ablation zone 105 defines a volume of outer adventitial tissue and adjacent perivascular space defined by inner zone diameter 105' and outer zone diameter 105". Renal nerves 14 and ganglia 24 included within the ablation zone 105 are subject to temperatures sufficient to cause necrosis of this renal nerve tissue and permanent termination of sympathetic renal nerve traffic along the renal artery 12.

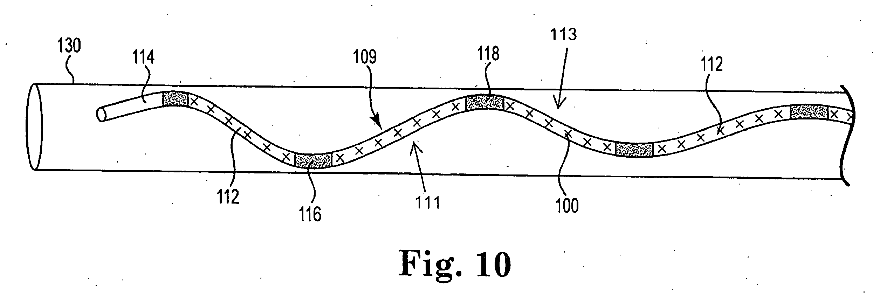

Figure 10 shows the distal end of an ablation catheter 100 which includes an ablation region 111 of a type previously described placed within a delivery sheath 130. In the embodiment shown in Figure 10, the ablation region 111 incorporates a flexible, self-deploying electrode spacing structure 113 in a somewhat compressed state. The delivery sheath 130 is preferably configured for advancement through a perivascular access location of a patient, through the patient's vascular system, and into the patient's renal artery via the aorta. In some embodiments, the delivery sheath 130 may define the sheath a guide catheter. Alternatively, the sheath 130 may define a conventional delivery sheath, and may be advanced through a lumen of a guide catheter which is used to access the patient's renal artery or at least an ostium of the renal artery.

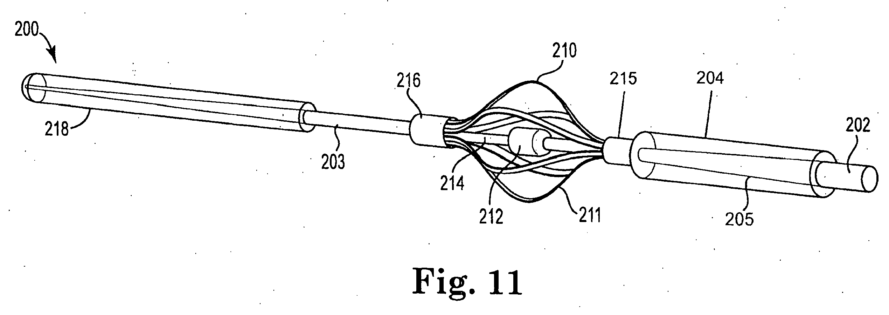

With reference to Figure 11, an ablation catheter 200 includes a flexible structure 210 that has a generally basket shape. The flexible structure 210 is preferably formed of a material that provides for self-expanding deployment. A relatively simple slotted-tube may be formed to define a self-expanding basket that surrounds an RF electrode 212. The basket structure may be formed from non-conductive material or may include a non-conductive coating to minimize any electrical effects. An external sheath, such as sheath 130 shown in Figure 10, may be advanced over the basket structure, causing the basket to collapse by sliding a distal mounting ring forward. The basket is preferably large enough to maintain positioning of the RF electrode 212 about 0.5 mm to about 1.0 mm from the artery's inner wall.

RF electrode 212 is shown mounted at a central location within the flexible structure 210, although non-centered (asymmetric) electrode positioning may be employed. The electrode 212 preferably includes a central bore through which a portion of a support core 214 passes. A proximal end of the flexible structure 210 is connected to an attachment ring 215, which is fixedly mounted to the support core 214. A distal end of the flexible structure 210 is connected to a sliding attachment ring 216. The sliding attachment ring 216 is configured for axial displacement in a proximal and distal direction relative to the fixed attachment ring 215.

Moving the sliding attachment ring 216 in a distal direction causes the flexible structure 210 to collapse. Moving the sliding attachment ring 216 in a proximal direction causes the flexible structure 210 to expand. The flexible structure 210 is fabricated as a self-deploying electrode spacing structure. For example the flexible structure 210 may be configured as a simple slotted-tube self-expanding basket. The flexible structure 210 is fabricated from a flexible material that requires external forces to cause expansion and contraction of the basket-shaped structure. An external sheath 204 may be used to facilitate collapsing and expanding of the flexible structure 210.

The external sheath 204 preferably has a lumen with a diameter slightly greater than a diameter of a proximal section 202 of the support core 214 and outer diameter of the attachment ring 215. A section 205 of the support core 214 between the proximal section 202 and the attachment ring 215 can have a tapering diameter. Collapsing of the flexible structure 210 is facilitated by advancing the external sheath 204 over the attachment ring 215, the flexible structure 210, and into abutment with sliding attachment ring 216. The sliding attachment ring 216 preferably has a diameter greater than that of the inner diameter of the external sheath 204.

When in abutment with sliding attachment ring 216 and in response to a distally directed force, the external sheath 204 forces forward (distally directed) movement of the sliding attachment ring 216 until the flexible structure 210 collapses into a non-deployed configuration. In another approach, the flexible structure 210 is collapsed by pulling it into the lumen of the external sheath 204, and the attachment ring 216 slides as needed. In one configuration, a guidewire tip 218 has a length such that its proximal end serves as a stop for the sliding attachment ring 216. In this configuration, the maximum distal travel distance of sliding attachment ring 216 is limited by the length of the guidewire tip 218. As indicated earlier, the external sheath 204 may be used in embodiments that include either a self-deploying or non-self-deploying flexible structure 210.

The distal end of the catheter apparatus shown in Figure 11 is directed towards the artery wall by a pre-formed bend in the catheter 200 or in an external sheath, use of a bent stylet, or by active articulation. The catheter 200 may be repositioned to move the electrode 212 axially and/or circumferentially for additional ablation treatment. The basket structure may be sized to span the renal artery. In this configuration, the RF electrode 212 is approximately centered in the artery and is held more than 1 mm from the artery wall. A circumferential "burn" can be achieved in this case. It is understood that more than one RF electrode 212 may be used, such as 2, 3, or 4 RF electrodes. It is further understood that more than one flexible structure 210 may be used, such as 2, 3, or 4, with at least one RF electrode 212 encompassed by each flexible structure 210.

The basket structure is sized to span the renal artery but is asymmetric, holding the RF electrode approximately 0.5-1.0 mm from one side of the artery wall. Multiple "burns" are used with repositioning of the catheter 200 to achieve the desired ablation of target tissue. This approach reduces power requirements and may limit unwanted heating of non-target tissue or blood. The basket structure 210 can be collapsed prior to repositioning the catheter 200 to minimize trauma to the artery wall.

As can be seen from Figures 4 and 5, the non-contact electrodes 212 of Figure 11 can provide some circumferential heating. By positioning more than one RF electrode 212 similarly away from the artery's inner wall at multiple circumferential locations, an entirely circumferential heating of the perivascular target region can be obtained, while maintaining the artery wall at safe temperatures. The electrodes 212 (typically 2, 3 or 4) can be energized simultaneously, or in sequence, or in a time-sharing manner to treat the entire target region efficiently, without requiring repositioning of the catheter 200 or electrode(s) 212. Other configurations of the basket structure 210 can be used, such as loops, round wires, braids, and so forth. Multiple short baskets 210 can be used, with at least one RF electrode 212 positioned in between, for example.

Various embodiments are directed to ablation devices that employ relatively inflexible or rigid electrode positioning arrangements that maintain a gap between one or more electrodes of the ablation device and body tissue, such as a vessel wall. Representative examples of such apparatuses include a catheter with bumps, protrusions, or other spacer elements, a catheter with electrode(s) positioned only on the lumen facing surface of the catheter, rigid portion(s) of a catheter that provide desired spacing from the vessel wall and orientation, and other structures that do not change shape between introduction and deployment.

Embodiments of the disclosure are directed to apparatuses for ablating target tissue of the body using a multiple electrode device that obviates the need for repositioning a single electrode. Embodiments of the disclosure are directed to apparatuses and methods for RF ablation of perivascular renal nerves for treatment of hypertension, using a multiple electrode device that obviates the need for repositioning a single electrode. Prior approaches have difficulty in reliably positioning an RF electrode at discrete ablation sites in the renal artery, and can require repeated repositioning and ablation cycles. Even with ablation of discrete locations, renal artery injury at these locations can occur due to local high temperatures resulting from high current density near the electrodes. To reduce thermal injury to the artery wall, renal nerve ablation approaches have been suggested that actively cool the renal artery during RF ablation, but such active cooling approaches are typically more complicated than passive cooling by the blood. Passive cooling by the blood, however, can be inadequate to protect the artery wall from injury.

Embodiments of the disclosure are directed to an ablation device that has a relatively simple wire or tube construction, with an ablation region being pre-set to take a shape with multiple short bends. When released in the renal artery by advancement of the device or by retraction of an external sheath, the ablation region of the device deploys to a polygonal spiral configuration. Shape-memory or superelastic slotted tube configurations can be utilized to provide a flexible self-deploying, self-centering multi-electrode structure.

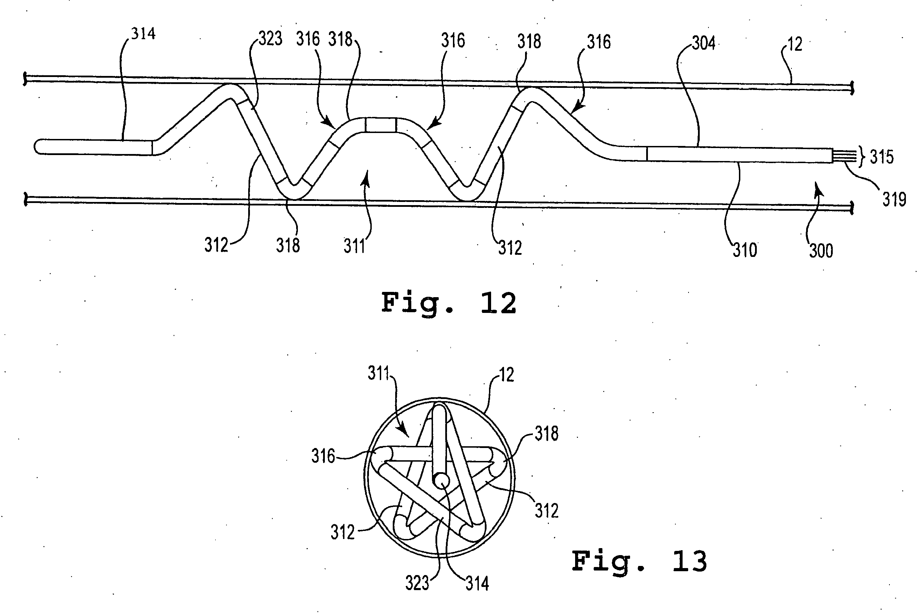

With reference to Figures 12 and 13, an ablation catheter 300 includes a flexible elongated element 310 having a length sufficient to access a target vessel 12 of the body, such as a patient's renal artery, relative to a percutaneous access location. The ablation catheter 300 has a distal end dimensioned for introduction into the renal artery 12. A self-deploying spacing structure 311 is provided at the distal end of the elongated element 310 and transformable between a low-profile introduction configuration and a larger-profile deployed configuration. The self-deploying spacing structure 311 comprises a multiplicity of pre-set bends 318 arranged to contact the wall of the renal artery 12 at discrete circumferential and axial locations when in the deployed configuration, as is best seen in Figure 13. Each of the pre-set bends 318 comprises an electrode surface 316. A section of non-conductive material 312 separates each electrode surface 316. A conductor arrangement 315 is coupled to the electrode surfaces 316 and extends along a length of the elongated element 310 to its proximal end. Preferably, each of the electrode surfaces 316 is coupled to separate conductor 319 to facilitate activation and deactivation of individual electrode surfaces 316.

The electrode arrangement shown in Figures 12 and 13 is configured as an angular electrode which is preferably constructed as a hollow structure with one or more fluid channels, but may be constructed as a solid serial electrode in some embodiments. The open configuration of the electrode arrangement provides for passive cooling of the electrode surfaces 316 by blood that flows through the vessel in which the electrode arrangement is deployed. A catheter shaft 304 that supports the electrode arrangement may include a heat sink shaft/tip to assist cooling. In this case, an elongated heat sink region of the tip can improve vessel wall cooling. The shaft 304 preferably has a flexible atraumatic tip 314 to assist in guiding the spacing structure 311 into the lumen or at least the ostium of the target vessel, such as a renal artery 12.

The self-deploying spacing structure 311 of the elongated element 310 shown in Figures 12 and 13 assumes a polygonal spiral configuration when deployed within a target vessel 12. In the deployed configuration, the pre-set bends 318 and, therefore, the electrode surfaces 316 are spaced circumferentially and axially apart from one another on the expanded spacing structure 311. According to one embodiment, the spacing structure 311 includes at least five of the pre-set bends 318 arranged at a predetermined pitch relative to one another to provide a pre-established relative axial and circumferential separation of ablation sites. The spacing structure 311 shown in Figure 13 includes five spanning chords 323 defining the non-conductive sections 312 of the elongated element 310. Each of the five spanning chords 323 includes at least one electrode surface 316 at each pre-set bend 318 situated between the electrically non-conductive sections 312. The number of pre-set bends 318 can vary, such as between 3 and 8 pre-set bends 318. In some embodiments, the electrode surfaces 316 are configured for unipolar operation. In other embodiments, pairs or combinations of the electrode surfaces 316 can be operated in a bipolar configuration.

In some embodiments, the elongated element 310 can include a central lumen and a multiplicity of layers of polymer tubing that electrically isolate the central lumen and an outer surface of the elongated element 310 other than regions defining the electrode surfaces 316. In other embodiments, the elongated element 310 defines a distal portion of a polymer tube of the elongated element 310. A shape-memory or superelastic metal shaping member may be situated in the polymer tube to define the spacing structure 311, which facilitates the spacing structure 311 assuming a polygonal spiral configuration within the renal artery when deployed. In further embodiments, the conductor arrangement 315 comprises an insulated slotted metal tube electrically coupled to the electrode surfaces 316. In other embodiments, insulated conductors can be passed through the central lumen of a tubular construction and attached to the electrode surfaces 316.

The spacing structure 311 preferably includes a shape-memory slotted tube or a superelastic slotted tube configured as a flexible self-deploying structure as shown in previously described Figure 8B. As discussed previously, various implementations may be used to provide desired bending characteristics of the pre-set bends 318 of the self-deploying spacing structure 311. Suitable hinges include those that bend easily in one plane, such hinges are referred to as orthotropic flexural stiffness hinges. A superelastic slotted tube represents a suitable structure for incorporating a hinge with desired orthotropic flexural stiffness characteristics. Other suitable hinges include orthotropic composite tubes, tubes with axial stiffeners, flat ribbons, bifilar arrangements of tubes, and multi-lumen tubing with lumens generally aligned with flexural plane.

In embodiments that do not incorporate a self-deploying spacing structure 311, an actuation wire may be coupled to the distal end of the catheter elongated element 310 to facilitate deployment and collapsing of the flexible spacing structure 311, such as by push-pull actuation as desired. In embodiments that incorporate a self-deploying flexible structure 313, inclusion of an actuation wire can be axially advanced and retracted to assist in collapsing and expansion of the flexible structure 113.

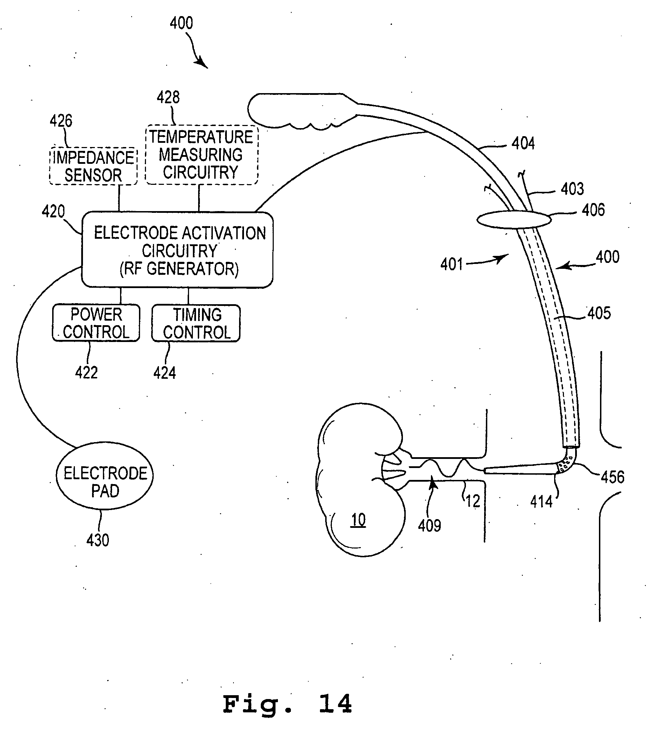

Figure 14 shows a representative RF renal therapy apparatus 400. The apparatus 400 illustrated in Figure 14 includes external electrode activation circuitry 420 which comprises power control circuitry 422 and timing control circuitry 424. The external electrode activation circuitry 420, which includes an RF generator, may be coupled to optional temperature measuring circuitry 428 and may be coupled to an optional impedance sensor 426. It is noted that some embodiments may not include temperature monitoring components, but use impedance to generally indicate temperature, or neither, using only time and power as a general indication of ablation progress.

The catheter 401 includes a shaft 404 that incorporates a lumen arrangement 405 configured for receiving a variety of components. A separate delivery sheath 403 may be used to facilitate deployment of the catheter 401 into the arterial system via a percutaneous access site 406 in the embodiment shown in Figure 14.

The RF generator of the external electrode activation circuitry 420 may include a pad electrode 430 that is configured to comfortably engage the patient's back or other portion of the body near the kidneys. Radiofrequency energy produced by the RF generator is coupled to an electrode arrangement 409 at the distal end of the catheter 401 by the conductor arrangement disposed in the lumen of the catheter's shaft 404. The electrode arrangement 409 is intended to represent any of the electrode arrangement embodiments described hereinabove.

Renal denervation therapy using the apparatus shown in Figure 14 is typically performed using the electrode arrangement 409 positioned within the renal artery 12 and the pad electrode 430 positioned on the patient's back, with the RF generator operating in a monopolar mode. In this implementation, the electrode arrangement 409 is configured for operation in a unipolar configuration. In other implementations, as previously discussed, the electrodes of the electrode arrangement 409 can be configured for operation in a bipolar configuration, in which case the pad electrode pad 430 is not needed.

The radiofrequency energy flows through the electrode arrangement 409 in accordance with a predetermined activation sequence (e.g., sequential or concurrent) causing ablative heating in the adjacent tissue of the renal artery. Two or more (or all) electrodes of the electrode arrangement 409 can be in electrical contact, such as by connecting insulated electrical conductors to two or more (or all) electrodes, and activating these electrodes simultaneously. Sets of electrodes can be in electrical contact, all electrodes of a given electrode set can be activated simultaneously, and individual electrode sets can be activated sequentially or concurrently. In general, when renal artery tissue temperatures rise above about 113° F (50° C), protein is permanently damaged (including those of renal nerve fibers). If heated over about 65° C, collagen denatures and tissue shrinks. If heated over about 65° C and up to 100° C, cell walls break and oil separates from water. Above about 100° C, tissue desiccates.

The electrode activation circuitry 420 is configured to control activation and deactivation of one or more electrodes of the electrode arrangement 409 in accordance with a predetermined energy delivery protocol and in response to signals received from temperature measuring circuitry 428. The electrode activation circuitry 420 controls radiofrequency energy delivered to the electrodes of the electrode arrangement 409 so as to maintain the current densities at a level sufficient to cause heating of the target tissue preferably to a temperature of at least about 55°C.

One or more temperature sensors are situated at the electrode arrangement 409 and provide for continuous monitoring of renal artery tissue temperatures, and RF generator power is automatically adjusted so that the target temperatures are achieved and maintained. An impedance sensor arrangement 426 may be used to measure and monitor electrical impedance during RF denervation therapy, and the power and timing of the RF generator 420 may be moderated based on the impedance measurements or a combination of impedance and temperature measurements.

Marker bands 414 can be placed on one or multiple parts of the ablation catheter 401, such as at the electrode arrangement 409, to enable visualization during the procedure. Other portions of the ablation catheter and/or delivery system, such as one or more portions of the shaft (e.g., at the hinge mechanism 456), may include a marker band 414. The marker bands 414 may be solid or split bands of platinum or other radiopaque metal, for example. Radiopaque materials are understood to be materials capable of producing a relatively bright or high-contrast image on a fluoroscopy screen or another imaging technique during a medical procedure. This relatively bright image aids the user in determining specific portions of the catheter 401, such as the tip of the catheter 401 or portions of the electrode arrangement 409, and the hinge 456, for example. A braid and/or electrodes of the catheter 401 can be radiopaque.

Various embodiments disclosed herein are generally described in the context of ablation of perivascular renal nerves for control of hypertension. It is understood, however, that embodiments of the disclosure have applicability in other contexts, such as performing ablation from within other vessels of the body, including other arteries, veins, and vasculature (e.g., cardiac and urinary vasculature and vessels), and other tissues of the body, including various organs.

It is to be understood that even though numerous characteristics of various embodiments have been set forth in the foregoing description, together with details of the structure and function of various embodiments, this detailed description is illustrative only, and changes may be made in detail, especially in matters of structure and arrangements of parts illustrated by the various embodiments to the full extent indicated by the broad general meaning of the terms in which the appended claims are expressed.

## Claims

1. An apparatus (100; 300; 400), comprising:
a flexible elongated element (110; 310) having a proximal end, a distal end, and a length;
a self-deploying structure (113; 313) transformable between a low-profile introduction configuration and a larger-profile deployed configuration;
an electrode arrangement (109; 409) supported by the self-deploying structure (113; 313) and configured to deliver ablative energy to target tissue adjacent a target vessel wall when the self-deploying structure (113; 313) is in the deployed configuration; and
a conductor arrangement (115; 315) coupled to the electrode arrangement (109; 409) and extending along the length of the elongated element (110; 310);
the apparatus **characterized in that** the self-deploying structure (113; 313) is provided at the distal end of the elongated element (110; 310) and comprises a plurality of pre-set bends (118; 318) arranged to contact a wall of the target vessel at discrete circumferential and axial locations when in the deployed configuration such that the self-deploying structure (113; 313) assumes a polygonal spiral configuration in the deployed configuration; and wherein:
the electrode arrangement (109; 409) comprises at least one electrode (112; 312); and
the self-deploying structure (113; 313) is configured to position the at least one electrode (112; 312) a predetermined distance away from the target vessel wall when in the deployed configuration.

2. The apparatus according to claim 1, wherein:
each of the pre-set bends (118; 318) comprises electrically insulating material; and
a segment of the elongated element (110; 310) between at least some of the pre-set bends comprises an electrode surface of the electrode arrangement.

3. The apparatus according to claim 1, wherein:
the electrode arrangement (109; 409) comprises at least one electrode (112; 312); and
the self-deploying structure (113; 313) is configured to position the at least one electrode at the target vessel wall while the ablative energy is delivered to the target tissue.

4. The apparatus according to claim 1, wherein at least some of the pre-set bends (118; 318) comprise an electrode surface.

5. The apparatus according to any of claims 1 through 4, wherein the elongated member (110; 310) comprises at least five of the pre-set bends (118; 318) arranged at a predetermined pitch relative to one another to provide a pre-established relative axial and circumferential separation of ablation sites.

6. The apparatus according to any of claims 1 through 5, wherein the self-deploying structure (113; 313) comprises a shape-memory slotted tube or a superelastic slotted tube configured as a flexible self-deploying structure.

7. The apparatus according to any of claims 1 through 5, wherein:
the elongated member (110; 310) comprises a polymeric tube; and
the conductor arrangement (115; 315) comprises an insulated slotted metal tube electrically coupled to the at least one electrode.

8. The apparatus according to any of claim 1 through claim 7, wherein:
the conductor arrangement (115; 315) comprises a plurality of conductors;
adjacent electrodes or electrode surfaces of the electrode arrangement (109; 409) are separated by electrically insulating material; and
each electrode or electrode surface is coupled to one of the plurality of conductors so that each electrode or electrode surface can be energized independently.

9. The apparatus according to any of claim 1 through claim 8, comprising an actuation wire (119) coupled to the distal end of the apparatus to facilitate deployment and collapsing of the self-deploying structure (113; 313).

10. The apparatus according to any of claim 1 through claim 9, wherein:
the conductor arrangement (115; 315) comprises a plurality of conductors;
the electrode arrangement (109; 409) comprises a plurality of electrodes electrically isolated from one another, one of the plurality of electrodes coupled to one of the plurality of conductors; and
the electrode arrangement (109; 409) is operable in a unipolar configuration or a bipolar configuration.

11. The apparatus according to any of claim 1 through claim 10, comprising one or more temperature sensors provided at the electrode arrangement.

12. The apparatus according to any of claim 1 through claim 11, comprising a sheath (130; 403) having a lumen dimensioned to receive the apparatus with the self-deploying structure in the low-profile introduction configuration, the sheath lumen further dimensioned for placement within the target vessel or ostium of the target vessel.

13. The apparatus according to any of claim 1 through claim 12, wherein:
the target vessel comprises a renal artery; and
the target tissue comprises perivascular renal nerve tissue adjacent the renal artery.

## Patentansprüche

1. Vorrichtung (100; 300; 400), mit:
einem flexiblen länglichen Element (110; 310), das ein proximales Ende, ein distales Ende und eine Länge aufweist;
einer selbstentfaltenden Struktur (113; 313), die zwischen einer Einführungskonfiguration mit niedrigem Profil und einer entfalteten Konfiguration mit größerem Profil umwandelbar ist;
einer Elektrodenanordnung (109; 409), die durch die selbstentfaltende Struktur (113; 313) gehalten wird und konfiguriert ist, ablative Energie an ein Zielgewebe benachbart einer Zielgefäßwand abzugeben, wenn sich die selbstentfaltende Struktur (113; 313) in der entfalteten Konfiguration befindet; und
einer Leiteranordnung (115; 315), die mit der Elektrodenanordnung (109; 409) gekoppelt ist und sich längs der Länge des länglichen Elements (110; 310) erstreckt;
wobei die Vorrichtung **dadurch gekennzeichnet ist, dass** die selbstentfaltende Struktur (113; 313) am distalen Ende des länglichen Elements (110; 310) vorgesehen ist und mehrere voreingestellte Biegungen (118; 318) aufweist, die so angeordnet sind, dass sie eine Wand des Zielgefäßes an diskreten Umfangs- und Axialstellen berühren, wenn sie sich in der entfalteten Konfiguration befindet, so dass die selbstentfaltende Struktur (113; 313) eine polygonale gewundene Konfiguration in der entfalteten Konfiguration annimmt; und wobei:
die Elektrodenanordnung (109; 409) mindestens eine Elektrode (112; 312) aufweist; und
die selbstentfaltende Struktur (113; 313) konfiguriert ist, die mindestens eine Elektrode (112; 312) in einem vorgegebenen Abstand von der Zielgefäßwand entfernt anzuordnen, wenn sie sich in der entfalteten Konfiguration befindet.

2. Vorrichtung nach Anspruch 1, wobei:
jede der voreingestellten Biegungen (118; 318) ein elektrisch isolierendes Material aufweist; und
ein Segment des länglichen Elements (110; 310) zwischen mindestens einigen der voreingestellten Biegungen eine Elektrodenfläche der Elektrodenanordnung aufweist.

3. Vorrichtung nach Anspruch 1, wobei:
die Elektrodenanordnung (109; 409) mindestens eine Elektrode (112; 312) aufweist; und
die selbstentfaltende Struktur (113; 313) konfiguriert ist, die mindestens eine Elektrode an der Zielgefäßwand anzuordnen, während die ablative Energie an das Zielgewebe abgegeben wird.

4. Vorrichtung nach Anspruch 1, wobei mindestens einige der voreingestellten Biegungen (118; 318) eine Elektrodenfläche aufweisen.

5. Vorrichtung nach einem der Ansprüche 1 bis 4, wobei das längliche Element (110; 310) mindestens fünf der voreingestellten Biegungen (118; 318) aufweist, die relativ zueinander in einer vorgegebenen Teilung angeordnet sind, um einen vorher festgelegten relativen Axial- und Umfangsabstand von Ablationsstellen bereitzustellen.

6. Vorrichtung nach einem der Ansprüche 1 bis 5, wobei die selbstentfaltende Struktur (113; 313) eine Formgedächtnis-Schlitzröhre oder eine superelastische Schlitzröhre aufweist, die als eine flexible selbstentfaltende Struktur konfiguriert ist.

7. Vorrichtung nach einem der Ansprüche 1 bis 5, wobei:
das längliche Element (110; 310) eine Polymerröhre aufweist; und
die Leiteranordnung (115; 315) eine isolierte Schlitzmetallröhre aufweist, die mit der mindestens einen Elektrode elektrisch gekoppelt ist.

8. Vorrichtung nach einem der Ansprüche 1 bis 7, wobei:
die Leiteranordnung (115; 315) mehrere Leiter aufweist;
benachbarte Elektroden oder Elektrodenflächen der Elektrodenanordnung (109; 409) durch ein elektrisch isolierendes Material getrennt sind; und
jede Elektrode oder Elektrodenfläche mit einem der mehreren Leiter gekoppelt ist, so dass jede Elektrode oder Elektrodenfläche unabhängig mit Energie versorgt werden kann.

9. Vorrichtung nach einem der Ansprüche 1 bis 8, die einen Betätigungsdraht (119) aufweist, der mit dem distalen Ende der Vorrichtung gekoppelt ist, um eine Entfaltung und Zusammenfaltung der selbstentfaltenden Struktur (113; 313) zu ermöglichen.

10. Vorrichtung nach einem der Ansprüche 1 bis 9, wobei:
die Leiteranordnung (115; 315) mehrere Leiter aufweist;
die Elektrodenanordnung (109; 409) mehrere Elektroden aufweist, die elektrisch voneinander isoliert sind, wobei eine der mehreren Elektroden mit einem der mehreren Leiter gekoppelt ist; und
die Elektrodenanordnung (109; 409) in einer unipolaren Konfiguration oder einer bipolaren Konfiguration betreibbar ist.

11. Vorrichtung nach einem der Ansprüche 1 bis 10, die einen oder mehrere Temperatursensoren aufweist, die an der Elektrodenanordnung vorgesehen sind.

12. Vorrichtung nach einem der Ansprüche 1 bis 11, die eine Hülle (130; 403) aufweist, die ein Lumen aufweist, das dimensioniert ist, die Vorrichtung mit der selbstentfaltenden Struktur in der Einführungskonfiguration mit niedrigem Profil aufzunehmen, wobei das Hüllenlumen ferner zur Anordnung innerhalb des Zielgefäßes oder des Ostiums des Zielgefäßes dimensioniert ist.

13. Vorrichtung nach einem der Ansprüche 1 bis 12, wobei
das Zielgefäß eine Nierenarterie aufweist; und
das Zielgewebe perivaskuläres Nierennervengewebe benachbart der Nierenarterie aufweist.

## Revendications

1. Dispositif (100 ; 300 ; 400), comprenant :
un élément allongé flexible (110 ; 310) ayant une extrémité proximale, une extrémité distale et une longueur ;
une structure auto-déployante (113 ; 313) pouvant passer d'une configuration d'insertion à profil réduit à une configuration de déploiement à profil étendu ;
une unité d'électrode (109 ; 409) supportée par la structure auto-déployante (113 ; 313) et prévue pour appliquer une énergie d'ablation à un tissu cible adjacent à une paroi de vaisseau cible quand la structure auto-déployante (113 ; 313) est en configuration de déploiement ; et
une agencement conducteur (115 ; 315) relié à l'unité d'électrode (109 ; 409) et s'étendant sur la longueur de l'élément allongé (110 ; 310) ;
ledit dispositif étant **caractérisé en ce que** la structure auto-déployante (113 ; 313) est prévue à l'extrémité distale de l'élément allongé (110 ; 310) et comprend une pluralité de coudes prédéfinis (118 ; 318) agencés pour contacter une paroi du vaisseau cible à des emplacements circonférentiels et axiaux discrets lorsque la structure est en configuration de déploiement, de sorte qu'en configuration de déploiement, ladite structure auto-déployante (113 ; 313) présente une configuration en spirale polygonale ; et où :
l'unité d'électrode (109 ; 409) comprend au moins une électrode (112 ; 312) ; et
la structure auto-déployante (113 ; 313) est prévue pour positionner ladite au moins une électrode (112 ; 312) à une distance définie de la paroi de vaisseau cible quand elle est en configuration de déploiement.

2. Dispositif selon la revendication 1, où :
chacun des coudes prédéfinis (118 ; 318) comprend un matériau électriquement isolant ; et
un segment de l'élément allongé (110 ; 310) entre au moins quelques-uns des coudes prédéfinis comprend une surface d'électrode de l'unité d'électrode.

3. Dispositif selon la revendication 1, où :
l'unité d'électrode (109 ; 409) comprend au moins une électrode (112 ; 312) ; et
la structure auto-déployante (113 ; 313) est prévue pour positionner ladite au moins une électrode sur la paroi de vaisseau cible, l'énergie d'ablation étant appliquée sur le tissu cible.

4. Dispositif selon la revendication 1, où au moins quelques-uns des coudes prédéfinis (118 ; 318) comprennent une surface d'électrode.

5. Dispositif selon l'une des revendications 1 à 4, où l'élément allongé (110 ; 310) comprend au moins cinq coudes prédéfinis (118 ; 318) disposés suivant un pas défini les uns par rapport aux autres afin de permettre une séparation axiale et circonférentielle relative préétablie entre sites d'ablation.

6. Dispositif selon l'une des revendications 1 à 5, où la structure auto-déployante (113 ; 313) comprend un tube perforé à mémoire de forme ou un tube perforé super-élastique configuré comme structure auto-déployante flexible.

7. Dispositif selon l'une des revendications 1 à 5, où :
l'élément allongé (110 ; 310) comprend un tube polymère ; et
l'agencement conducteur (115 ; 315) comprend un tube métallique perforé isolé, électriquement relié à ladite au moins une électrode.

8. Dispositif selon l'une des revendications 1 à 7, où :
l'agencement conducteur (115 ; 315) comprend une pluralité de conducteurs ;
des électrodes ou des surfaces d'électrode adjacentes de l'unité d'électrode (109 ; 409) sont séparées par un matériau isolant électrique ; et
chaque électrode ou surface d'électrode est reliée à un des conducteurs de la pluralité de conducteurs, de sorte que chaque électrode ou surface d'électrode peut être excitée indépendamment.

9. Dispositif selon l'une des revendications 1 à 8, comprenant un fil d'actionnement (119) relié à l'extrémité distale du dispositif pour faciliter le déploiement et la contraction de la structure auto-déployante (113 ; 313).

10. Dispositif selon l'une des revendications 1 à 9, où :
l'agencement conducteur (115 ; 315) comprend une pluralité de conducteurs ;
l'unité d'électrode (109 ; 409) comprend une pluralité d'électrodes électriquement isolées les unes des autres, une électrode de la pluralité d'électrodes étant reliée à un des conducteurs de la pluralité de conducteurs ; et
l'unité d'électrode (109 ; 409) peut être mise en oeuvre dans une configuration unipolaire ou une configuration bipolaire.

11. Dispositif selon l'une des revendications 1 à 10, comprenant un ou plusieurs capteurs de température prévus sur l'unité d'électrode.

12. Dispositif selon l'une des revendications 1 à 11, comprenant une gaine (130 ; 403) présentant une lumière dimensionnée pour recevoir le dispositif avec la structure auto-déployante en configuration d'insertion à profil réduit, la lumière de la gaine étant en outre dimensionnée pour une disposition à l'intérieur du vaisseau cible ou de l'ostium du vaisseau cible.

13. Dispositif selon l'une des revendications 1 à 12, où :
le vaisseau cible comprend une artère rénale ; et
le tissu cible comprend un tissu nerveux rénal périvasculaire adjacent à l'artère rénale.
